(19) Europäisches Patentamt
European Patent Office
Office européen des brevets

(11) Numéro de publication: **0 025 619**
**B1**

(12) **FASCICULE DE BREVET EUROPEEN**

(45) Date de publication du fascicule du brevet: **13.04.83**

(51) Int. Cl.³: **C 07 C 65/34**, C 07 C 51/60

(21) Numéro de dépôt: **80200817.7**

(22) Date de dépôt: **01.09.80**

(54) **Chlorures d'acyles et procédé pour leur fabrication.**

(30) Priorité: **13.09.79 FR 7923066**

(43) Date de publication de la demande:
**25.03.81 Bulletin 81/12**

(45) Mention de la délivrance du brevet:
**13.04.83 Bulletin 83/15**

(84) Etats contractants désignés:
**AT BE CH DE FR GB IT LI LU NL SE**

(56) Documents cités:
**US - A - 2 629 737**

**BULLETIN DES SOCIETES CHIMIQUES BELGES, vol. 71, 1962, Société chimique de Belgique BE J. GOLDSTEIN-GOLAIRE et al.: "Etude de la structure et des propriétés du chlorure d'ortho mésitoylbenzoyle et de quelques homologues", pages 159—176**

**JOURNAL OF THE CHEMICAL SOCIETY, PERKIN TRANSACTIONS II, 1973, (6) Londres GB M. VIVEKANANDABHATT et al.: "Aspects of Tautomerism. Part IV. A Kinetic Study of the Dimerization Reaction of Pseudo-acid Chlorides", pages 1158—1160**

(73) Titulaire: **INTEROX Société anonyme dite:
Rue du Prince Albert, 33
B-1050 Bruxelles (BE)**

(72) Inventeur: **Bouillet, Edmond
Avenue de l'Amarante, 6
B-1020 Bruxelles (BE)**
Inventeur: **Schoemans, Jean
Boulevard Reyers, 167 - Boîte 24
B-1040 Bruxelles (BE)**

(74) Mandataire: **Eischen, Roland
Solvay & Cie Département de la Propriété Industrielle Rue de Ransbeek 310
B-1120 Bruxelles (BE)**

(56) Documents cités:
**JOURNAL OF ORGANIC CHEMISTRY, vol. X, 1945 Baltimore US R.C. FUSON et al.: "Synthesis of o-diaroylbenzenes and 1,2-diaroyl-cyclohexanes", pages 55—61**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 025 619 B1

# Chlorures d'acyles et procédé pour leur fabrication

La présente invention concerne de nouveaux chlorures d'acyles ainsi que des procédés pour leur fabrication.

Les chlorures d'acyles sont des réactifs fréquemment utilisés en chimie fine pour les synthèses organiques, notamment dans les réactions de type Friedel-Crafts d'alkylation ou d'arylation.

Les essais de préparation de chlorures d'acides o-(benzoyl) benzoïques, substitués ou non, à structure ouverte ont été jusqu'à présent presque tous infructueux. Ainsi, lorsqu'on fait réagir avec le chlorure de thionyle les acides o-(benzoyl) benzoïques non substitués, comme décrit dans le brevet US—A—2 629 737 (G.D. Searle & Co), ou substitués en 2'- ou en 4'- par des groupes méthyles, on obtient des pseudo-chlorures d'acides ayant une structure fermée du type 3-chloro, 3-phénylphtalide (M. Vivekananda Bhatt et coll., J. Chem. Soc., Perkin II, 1973, *6*, p. 1158 à 1160) (J. Golstein-Golaire et coll., Bull. Soc. Chim. Belg., 1962, *71*, p. 159 à 176). Pour pouvoir obtenir un vrai chlorure d'acide à structure ouverte, il faut mettre en oeuvre un acide o-(aroyl) benzoïque substitué simultanément en 2'- et en 6'- par un groupe méthyle. Sit on fait réagir le chlorure de phtaloyle avec le composé aromatique correspondant, on obtient un o-(diaroyl)benzène (R. Fuson et coll., J. Org. Chem., 1945, X, p. 55 à 61).

La présente invention vise à fournir de nouveaux chlorures d'acides o-(benzoyl)benzoïques à structure ouverte qui conviennent bien comme intermédiaires de synthèse.

L'invention concerne à cet effet des chlorures d'acyles qui répondent à la formule

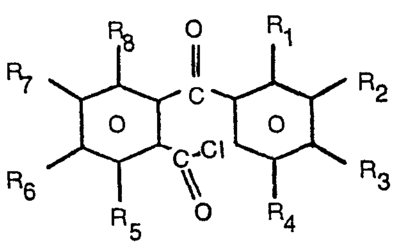

(I)

où les substituants $R_1$, $R_2$, $R_4$, $R_5$, $R_6$, $R_7$ et $R_8$ représentent un atome d'hydrogène ou un groupement carboné contenant de 1 à 12 atomes de carbone, $R_1$, $R_2$, $R_4$, $R_5$, $R_6$, $R_7$ et $R_8$ pouvant être identiques ou différents, et où $R_3$ représente un groupement carboné contenant au moins deux atomes de carbone, et où les groupements carbonés précités sont des groupes alkyles, alkényles, aryles, alkylaryles, alkénylaryles, aralkyles, aralkényles, cycloalkyles ou cycloalkényles pouvant contenir dans leur structure au moins un hétéroatome constitué par un halogène, l'oxygène, l'azote ou le soufre.

Les groupements carbonés précités peuvent également constituer deux à deux ($R_1$—$R_2$, $R_2$—$R_3$, $R_3$—$R_4$, $R_5$—$R_6$, $R_6$—$R_7$, et/ou $R_7$—$R_8$) une ou plusieurs chaînes carbonées uniques.

Parmi les chlorures d'acyles selon l'invention, ceux pour lesquels le substituant $R_3$ représente un groupement carboné contenant de 2 à 8 atomes de carbone et de préférence un groupement alkyle contenant de 2 à 8 atomes de carbone sont préférés.

Par ailleurs, les chlorures d'acyles répondant à la formule I où les substituants $R_5$ et $R_7$ représentent l'hydrogène sont également préférés. Les chlorures d'acyles où les substituants $R_2$, $R_4$, $R_5$ et $R_7$ représentent un atome d'hydrogène et qui répondent à la formule

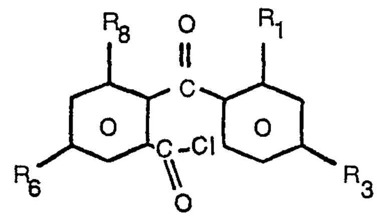

(II)

sont particulièrement préférés.

Les chlorures d'acyles les plus préférés selon l'invention sont ceux qui répondent à la formule

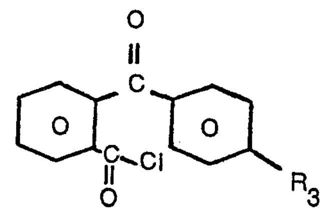

(III)

où $R_3$ représent groupement alkyle contenant de 2 à 8 atomes de carbone. Parmi ceux-ci figurent les chlorures d'acide ortho-(4'-propylbenzoyl)-, -(4'-isopropylbenzoyl)-, -(4'-butylbenzoyl)-, -(4'-isobutylbenzoyl)-, -(4'-tert-butylbenzoyl)-, -(4'-pentylbenzoyl)-, -(4'-sec-amylbenzoyl)- et -(4'-tert-amylbenzoyl)-benzoique.

Les chlorures d'acyles selon l'invention peuvent être obtenus selons divers procédés.

Un procédé pour la fabrication de chlorures d'acyles de l'invention consiste à faire réagir l'acide ortho-benzoylbenzoïque substitué correspondant ou éventuellement un de ses sels avec un agent chlorant. L'agent chlorant est en général choisi parmi le trichlorure de phosphore, le pentachlorure de phosphore, l'oxychlorure de phosphore, le chlorure de thionyle, le chlorure d'oxalyle, le phosgène et les chlorures d'acides sulfoniques tels que le chlorure d'acide benzène sulfonique. De bon résultats ont été obtenus en faisant réagir les acides orthobenzoylbenzoïques substitués avec les chlorures et oxychlorures de phosphore et le chlorure de thionyle.

Le rapport molaire entre l'agent chlorant et l'acide orthobenzoylbenzoïque substitué ou son sel est en général compris entre 0,1 et 10. De bons résultats ont été obtenus avec des rapports molaires compris entre 0,9 et 1,5.

La réaction se fait en général en phase liquide. Elle peut se faire en présence ou en l'absence de solvant. En général, on opère en présence d'un solvant. Les solvants mis en oeuvre sont des solvants inertes dans les conditions de réaction. De préférence, on utilise des solvants capables de solubiliser l'acide orthobenzoylbenzoïque et l'agent chlorant.

Les solvants sont en général choisis parmi les hydrocarbures et leurs dérivés substitués contenant de 1 à 12 atomes de carbone. On utilise le plus souvent des hydrocarbures substitués. Les substituants sont le plus souvent choisis parmi les atomes d'halogène et plus particulièrement de chlore, et les amides. De bons résultats ont été obtenus avec des hydrocarbures chlorés contenant de 1 à 8 atomes de carbone, tels que le monochlorobenzène et le tétrachlorure de carbone.

Le mélange réactionnel contient en général de 30 à 98% et de préférence de 50 à 95% en poids de solvant.

La température de réaction est en général comprise entre 250 et 350 K et le plus souvent 260 et 320 K.

Au cours de la réaction, on veille de préférence à maintenir le mélange réactionnel à l'état substantiellement anhydre. A cette fin, on opère en général sous atmosphère inerte; on peut ainsi utiliser de l'azote ou des gaz rares.

Le chlorure d'acyle peut être séparé des autres constituants du mélange réactionnel notamment par évaporation de ces derniers. Cette évaporation se fait en général sous pression réduite, en général comprise entre 0,001 et 0,5 bar. On opère en général à des températures comprises entre 300 et 400 K.

Un autre procédé pour la fabrication des chlorures d'acyles selon l'invention consiste à faire réagir le chlorure d'acide orthophtalique éventuellement substitué correspondant avec le composé benzénique substitué correspondant. Ce procédé convient plus particulièrement pour la préparation de chlorures d'acyles répondant aux formules générales (II) et (III) ci-avant.

Les deux réactifs sont en général mis en oeuvre dans des proportions voisines de la stoechiométrie. De bons résultats ont été obtenus en utilisant un rapport molaire chlorure d'acide orthophtalique sur composé benzénique compris entre 0,8 et 1,2.

La réaction est réalisée en général en présence d'un catalyseur. Comme catalyseur, on utilise en général des catalyseurs du type Friedel-Crafts et leurs mélanges. Des exemples de ces catalyseurs sont donnés dans le livre Friedel-Crafts and Related Reactions de G.A. Olah, volume I, 1963, Interscience Publ. On peut ainsi utiliser des acides Lewis et des acides de Brönsted. On utilise le plus souvent des acides de Lewis non protoniques. Parmi ceux-ci les halogénures et plus particulièrement les chlorures métalliques conviennent bien. De bons résultats ont été obtenus avec le chlorure d'aluminium.

Ces catalyseurs sont en général mis en oeuvre en quantités relativement importantes. Le plus souvent, on utilise de 0,1 à 20 mol de catalyseur par mol de chlorure d'acide orthophtalique éventuellement substitué. De bons résultats ont été obtenus en mettant en oeuvre au moins 1 mol de catalyseur par mol de chlorure d'acide orthophtalique. Les meilleurs résultats ont été obtenus en mettant en oeuvre de 2 à 10 mol de catalyseur par mol de chlorure d'acide orthophtalique.

Le catalyseur est de préférence introduit dans le réacteur, avant les autres réactifs, soit tel quel, soit sous forme de solution dans le solvant de la réaction. On introduit ensuite le composé benzénique et enfin le chlorure d'acide orthophtalique. L'ordre inverse d'introduction des réactifs peut également être utilisé.

La réaction est effectuée en général en phase liquide. Elle peut se faire en présence ou en l'absence d'un solvant. Les solvants mis en oeuvre sont des solvents inertes dans les conditions de réaction. De préférence, ils sont capables de solubiliser le chlorure d'acide orthophtalique et le composé benzénique, ainsi que, le catalyseur mis en oeuvre. On peut utiliser un solvant unique ou un mélange de solvants.

Les solvants sont en général choisis parmi les hydrocarbures substitués ou non. Ces hydrocarbures contiennent en général de 1 à 12 atomes et de préférence de 1 à 8 atomes de carbone. Ils peuvent être de types divers et comporter des groupes tels que des groupes alkyles, alkényles, cycloalkyles, cycloalkényles, aryles, aralkyles, aralkényles, alkylaryles et alkénylaryles et des hétérocycles. Leurs substituants éventuels peuvent être de natures très diverses; ils sont en général choisis parmi les atomes d'halogène et de soufre et les groupes nitro, sulfone, sulfoxyde, éther et thioéther. En général, on met en oeuvre des hydrocarbures substitués. Les hydrocarbures halogénés et plus particulièrement les hydrocarbures chlorés, plus particulièrement ceux qui contiennent de 1 à 7 atomes de carbone, ainsi que le sulfure de carbone conviennent bien. De bons résultats ont été obtenus avec le tétrachlorométhane, le trichlorométhane, le dichlorométhane, le 1,2-dichloroéthane, le 1,2-dichloroéthylène, le trichloréthylène et le perchloroéthylène.

Les solvants éventuels peuvent être mis en oeuvre en quantitiés variables. En général, lorsqu'on met en oeuvre un solvant, le mélange réactionnel contient de 50 à 95% en poids de solvant.

La température de réaction est en général relativement basse et ne dépasse le plus souvent pas 350 K. De bons résultats ont été obtenus à des températures comprises entre 250 et 300 K. Aux températures supérieures à

350 K, on observe une isomérisation en un dérivé du type phénylphtalide.

Les chlorures d'acyles selon l'invention peuvent être utilisés comme intermédiaires de synthèse, dans des réactions de condensation du type Friedel-Crafts. Ils peuvent ainsi être utilisés dans la synthèse des colorants.

Afin d'illustrer l'invention, sans pour autant en limiter le portée, on donne ci-après des exemples de fabrication de chlorures d'acides ortho-benzoylbenzoïques substitués.

Exemple 1

Dans un réacteur pourvu d'un agitateur et contenant une suspension de particules de $PCl_5$ dispersés du monochlorobenzène sec, on introduit goutte à goutte une solution d'acide ortho-(4'-éthylbenzoyl)-benzoïque dans du monochlorobenzène. Le rapport molaire entre le chlorure de phosphore et l'acide est amené à 1,1. Les quantités de réactifs sont mises en oeuvre de manière à obtenir une solution contenant en théorie 140 g par kg de chlorure d'acyle selon l'invention.

La température dans le réacteur est maintenue entre 274 et 275 K. La durée d'introduction est de 2 h environ. On opère sous atmosphère d'azote sec.

Après 2 h de réaction, on évapore la solution sous vide (pression de 0,033 bar) à une température comprise entre 353 et 373 K. On élimine ainsi le monochlorobenzène, l'oxychlorure de phosphore et le chlorure d'hydrogène.

On obtient ainsi du chlorure d'acide ortho-(4'-éthylbenzoyl)-benzoïque à 92%.

Exemple 2

On opère comme indiqué à l'exemple 1. On met en oeuvre une solution d'acide ortho-(4'-tert-butylbenzoyl)-benzoïque dans du monochlorobenzène.

On obtient ainsi du chlorure d'acide ortho-(4'-tert-butylbenzoyl)-benzoïque à 94%.

Exemple 3

On opère comme indiqué à l'exemple 1. On met en oeuvre une solution d'un mélange d'acides ortho-(4'-sec- et 4'-tert-amylbenzoyl)-benzoïques dans du monochlorobenzène.

On obtient ainsi un mélange de chlorures d'acides ortho-(4'-sec- et 4'-tert-amylbenzoyl)-benzoïques à environ 90%.

Exemple 4

Dans un réacteur muni d'un agitateur et d'un réfrigérant et maintenu sous une atmosphère d'azote sec, on introduit une suspension de 126 g de chlorure d'aluminium broyé dans 0,200 l de 1,2-dichloréthane.

On introduit ensuite progressivement, à la température ambiante 100 g de chlorure de phtaloyle.

Après dissolution complète du chlorure d'aluminium, on amène le mélange réactionnel à une température de 281—283 K et on introduit progressivement 0,45 mol d'éthylbenzène.

On obtient environ 35% de chlorure d'acide ortho-(4'-éthylbenzoyl)-benzoïque.

**Revendications pour les états contractants: BE CH DE FR GB IT LI LU NL SE**

1. Chlorures d'acyles caractérisés en ce qu'ils répondent à la formule

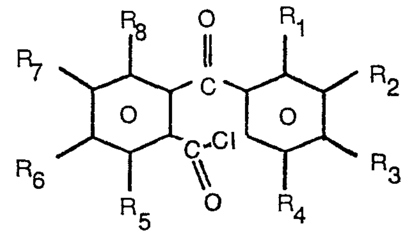

(I)

où les substituants $R_1$, $R_2$, $R_4$, $R_5$, $R_6$, $R_7$ et $R_8$ représentent un atome d'hydrogène ou un groupement carboné contenant de 1 à 12 atomes de carbone, $R_1$, $R_2$, $R_4$, $R_5$, $R_6$, $R_7$ et $R_8$ pouvant être identiques ou différents, où $R_3$ représente un groupement carboné contenant au moins deux atomes de carbone, et où les groupements carbonés sont des groupes alkyles, alkényles, aryles, alkylaryles, alkénylaryles, aralkyles, aralkényles, cycloalkyles ou cycloalkényles pouvant contenir dans leur structure au moins un hétéroatome constitué par un halogène, l'oxygène, l'azote ou le soufre.

2. Chlorures d'acyles selon la revendication 1, caractérisés en ce que $R_2$, $R_4$, $R_5$ et $R_7$ représentent un atome d'hydrogène.

3. Chlorures d'acyles selon la revendication 2 caractérisés en ce que $R_1$, $R_6$ et $R_8$ représentent également un atome d'hydrogène.

4. Chlorures d'acyles selon l'une quelconque des revendications 1 à 3, caractérisés en ce que $R_3$ représente un groupe alkyle contenant de 2 à 8 atomes de carbone.

5. Procédé pour la fabrication de chlorures d'acyles selon l'une quelconque des revendications 1 à 4, caractérisé en ce qu'on fait réagir l'acide ortho-benzoyl-benzoïque substitué correspondant avec un agent chlorant.

6. Procédé selon la revendication 5, caractérisé en ce que l'on met en oeuvre un agent chlorant choisi parmi les chlorures et oxychlorures de phosphore et le chlorure de thionyle.

7. Procédé selon la revendication 5 ou 6, caractérisé en ce qu'on effectue la réaction à une température comprise entre 250 et 350 K.

8. Procédé pour la fabrication de chlorures d'acyles selon l'une quelconque des revendications 1 à 4, caractérisé en ce qu'on fait réagir le chlorure d'acide ortho-phtalique éventuellement substitué correspondant avec le composé benzénique substitué correspondant en présence d'un catalyseur du type Friedel-Crafts.

9. Procédé selon la revendication 8,

caractérisé en ce qu'on utilise du chlorure d'aluminium comme catalyseur.

10. Procédé selon l'une ou l'autre des revendications 9 et 10, caractérisé en ce qu'on utilise au moins 1 mol de catalyseur par mol de chlorure d'acide ortho-phtalique.

11. Procédé selon l'une quelconque des revendications 8 à 10, caractérisé en ce qu'on opère à une température ne dépassant pas 350 K.

**Revendications pour l'état contractant: AT**

1. Procédé pour la fabrication de chlorures d'acyles répondant à la formule

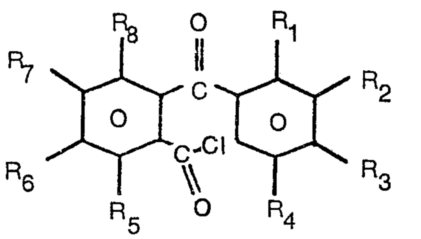

(I)

où les substituants $R_1$, $R_2$, $R_4$, $R_5$, $R_6$, $R_7$ et $R_8$ représentent un atome d'hydrogène ou un groupement carboné contenant de 1 à 12 atomes de carbone, $R_1$, $R_2$,'$R_4$, $R_5$, $R_6$, $R_7$ et $R_8$ pouvant être identiques ou différents, où $R_3$ représente un groupement carboné contenant au moins deux atomes de carbone, et où les groupements carbonés sont des groupes alkyles, alkényles, aryles, alkylaryles, alkénylaryles, aralkyles, aralkényles, cycloalkyles ou cycloalkényles pouvant contenir dans leur structure au moins un hétéroatome constitué par un halogène, l'oxygène, l'azote ou le soufre, caractérisé en ce qu'on fait réagir l'acide ortho-benzoyl-benzoïque substitué correspondant avec un agent chlorant.

2. Procédé selon la revendication 1, caractérisé en ce que l'on met en oeuvre un agent chlorant choisi parmi les chlorures et oxychlorures de phosphore et le chlorure de thionyle.

3. Procédé selon la revendication 1 ou 2, caractérisé en ce qu'on effectue la réaction à une température comprise entre 250 et 350 K.

4. Procédé pour la fabrication de chlorures d'acyles répondant à la formule

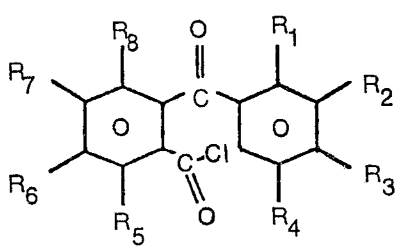

(I)

où les substituants $R_1$, $R_2$, $R_4$, $R_5$, $R_6$, $R_7$ et $R_8$ représentent un atome d'hydrogène ou un groupement carboné contenant de 1 à 12 atomes de carbone, $R_1$, $R_2$, $R_4$, $R_5$, $R_6$, $R_7$ et $R_8$ pouvant être identiques ou différents, où $R_3$ représente un groupement carboné contenant au moins deux atomes de carbone, et où les groupements carbonés sont des groupes alkyles, alkényles, aryles, alkylaryles, alkénylaryles, aralkyles, aralkényles, cycloalkyles ou cycloalkényles pouvant contenir dans leur structure au moins un hétéroatome constitué par un halogène, l'oxygène, l'azote ou le soufre, caractérisé en ce qu'on fait réagir le chlorure d'acide ortho-phtalique éventuellement substitué correspondant avec le composé benzénique substitué correspondant en présence d'un catalyseur du type Friedel-Crafts.

5. Procédé selon la revendication 4, caractérisé en ce qu'on utilise du chlorure d'aluminium comme catalyseur.

6. Procédé selon la revendication 4 ou 5, caractérisé en ce qu'on utilise au moins 1 mol de catalyseur par mol de chlorure d'acide ortho-phtalique.

7. Procédé selon l'une quelconque des revendications 4 à 6, caractérisé en ce qu'on opère à une température ne dépassant pas 350 K.

8. Procédé selon l'une quelconque des revendications 1 à 7, caractérisé en ce que $R_2$, $R_4$, $R_5$ et $R_7$ représentent un atome d'hydrogène.

9. Procédé selon l'une quelconque des revendications 1 à 8, caractérisé en ce que $R_1$, $R_6$ et $R_8$ représentent également un atome d'hydrogène.

10. Procédé selon l'une quelconque des revendications 1 à 9, caractérisé en ce que $R_3$ représente un groupe alkyle contenant de 2 à 8 atomes de carbone.

**Patentansprüche für die Vertragsstaaten: BE CH DE FR GB IT LI LU NL SE**

1. Acylchloride, dadurch gekennzeichnet, dass sie der Formel

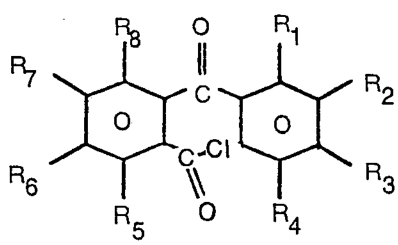

(I)

entsprechen, worin die Substituenten $R_1$, $R_2$, $R_4$, $R_5$, $R_6$, $R_7$ und $R_8$ ein Wasserstoffatom oder eine Kohlenstoff enthaltende Gruppe mit 1 bis 12 Kohlenstoffatomen bedeuten, wobei $R_1$, $R_2$, $R_4$, $R_5$, $R_6$, $R_7$ und $R_8$ gleich oder verschieden sein können, worin $R_3$ eine Kohlenstoffhaltige Gruppe mit mindestens 2 Kohlenstoffatomen darstellt, und worin die Kohlenstoff-haltigen Gruppen Alkylgruppen, Alkenylgruppen, Arylgruppen, Alkylarylgruppen, Alkenylarylgruppen, Aralkylgruppen, Aralkenylgruppen, Cycloalkylgruppen oder Cycloalkenylgruppen sind, die in

ihrer Struktur wenigstens ein Heteroatom, bestehend aus einem Halogen, Sauerstoff, Stickstoff oder Schwefel, enthalten können.

2. Acylchloride nach Anspruch 1, dadurch gekennzeichnet, dass $R_2$, $R_4$, $R_5$ und $R_7$ ein Wasserstoffatom bedeuten.

3. Acylchloride nach Anspruch 2, dadurch gekennzeichnet, dass $R_1$, $R_6$ und $R_8$ gleichfalls ein Wasserstoffatom bedeuten.

4. Acylchloride nach einem der Ansprüche 1 bis 3, dadurch gekennzeichnet, dass $R_3$ eine 2 bis 8 Kohlenstoffatome aufweisende Alkylgruppe darstellt.

5. Verfahren zur Herstellung der Acylchloride gemäss einem der Ansprüche 1 bis 4, dadurch gekennzeichnet, dass man eine entsprechend substituierte o-Benzoyl-benzoesäure mit einem Chlorierungsmittel zur Reaktion bringt.

6. Verfahren nach Anspruch 5, dadurch gekennzeichnet, dass man ein unter den Chloriden und Oxychloriden von Phosphor und Thionylchlorid ausgewähltes Chlorierungsmittel einsetzt.

7. Verfahren nach Anspruch 5 oder 6, dadurch gekennzeichnet, dass man die Umsetzung bei einer Temperatur zwischen 250 und 350 K durchführt.

8. Verfahren zur Herstellung von Acylchloriden gemäss einem der Ansprüche 1 bis 4, dadurch gekennzeichnet, dass mas das gegebenenfalls entsprechend substituierte o-Phthalsäurechlorid mit der entsprechend substituierten Benzolverbindung in Gegenwart eines Katalysators vom Friedel-Crafts-Typ umsetzt.

9. Verfahren nach Anspruch 8, dadurch gekennzeichnet, dass mas als Katalysator Aluminiumchlorid verwendet.

10. Verfahren nach dem einen oder dem anderen der Ansprüche 8 und 9, dadurch gekennzeichnet, dass man mindestens ein Mol Katalysator je Mol o-Phthalsäurechlorid anwendet.

11. Verfahren nach einem der Ansprüche 8 bis 10, dadurch gekennzeichnet, dass man bei einer 350 K nicht überschreitenden Temperatur arbeitet.

**Patentansprüche für den Vertragsstaat: AT**

1. Verfahren zur Herstellung von Acylchloriden entsprechend der Formel

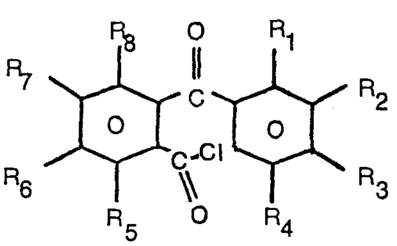

, (I)

worin die Substituenten $R_1$, $R_2$, $R_4$, $R_6$, $R_7$ und $R_8$ ein Wasserstoffatom oder eine Kohlenstoff enthaltende Gruppe mit 1 bis 12 Kohlenstoffatomen bedeuten, wobei $R_1$, $R_2$, $R_4$, $R_5$, $R_6$, $R_7$ und $R_8$ gleich oder verschieden sein können, worin $R_3$ eine Kohlenstoffhaltige Gruppe mit mindestens Kohlenstoffatomen darstellt, und worin die Kohlenstoff-haltigen Gruppen Alkylgruppen, Alkenylgruppen, Arylgruppen, Alkylarylgruppen, Alkenylarylgruppen, Aralkylgruppen, Aralkenylgruppen, Cycloalkylgruppen oder Cycloalkenylgruppen sind, die in ihrer Struktur wenigstens ein Heteroatom, bestehend aus einem Halogen, Sauerstoff, Stickstoff oder Schwefel, enthalten können, dadurch gekennzeichnet, dass man eine entsprechend substituierte o-Benzoyl-benzoesäure mit einem Chlorierungsmittel umsetzt.

2. Verfahren nach Anspruch 1, dadurch gekennzeichnet, dass man ein unter den Chloriden und Oxychloriden von Phosphor und Thionylchlorid ausgewähltes Chlorierungsmittel einsetzt.

3. Verfahren nach Anspruch 1 oder 2, dadurch gekennzeichnet, dass man die Umsetzung bei einer Temperatur zwischen 250 und 350 K durchführt.

4. Verfahren zur Herstellung von Acylchloriden entsprechend der Formel

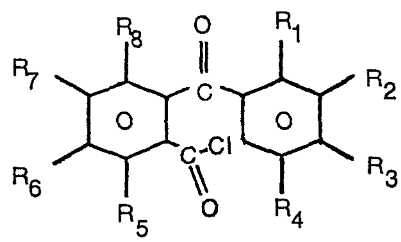

, (I)

worin die Substituenten $R_1$, $R_2$, $R_4$, $R_5$, $R_6$, $R_7$ und $R_8$ ein Wasserstoffatom oder eine Kohlenstoff enthaltende Gruppe mit 1 bis 12 Kohlenstoffatomen bedeuten, wobei $R_1$, $R_2$, $R_4$, $R_5$, $R_6$, $R_7$ und $R_8$ gleich oder verschieden sein können, worin $R_3$ eine Kohlenstoff-haltige Gruppe mit mindestens 2 Kohlenstoffatomen darstellt, und worin die Kohlenstoff-haltigen Gruppen Alkylgruppen, Alkenylgruppen, Arylgruppen, Alkylarylgruppen, Alkenylarylgruppen, Aralkylgruppen, Aralkenylgruppen, Cycloalkylgruppen oder Cycloalkenylgruppen sind, die in ihrer Struktur wenigstens ein Heteroatom, bestehend aus einem Halogen, Sauerstoff, Stickstoff oder Schwefel, enthalten können, dadurch gekennzeichnet, dass mann das gegebenenfalls entsprechend substituierte o-Phthalsäurechlorid mit der entsprechend substituierten Benzolverbindung in Gegenwart eines Katalysators vom Friedel-Crafts-Typ umsetzt.

5. Verfahren nach Anspruch 4, dadurch gekennzeichnet, dass man als Katalysator Aluminiumchlorid verwendet.

6. Verfahren nach Anspruch 4 oder 5, dadurch gekennzeichnet, dass man mindestens ein Mol Katalysator je Mol o-Phthalsäurechlorid anwendet.

7. Verfahren nach einem der Ansprüche 4 bis 6, dadurch gekennzeichnet, dass man bei einer 350 K nicht überschreitenden Temperatur arbeitet.

8. Verfahren nach einem der Ansprüche 1 bis 7, dadurch gekennzeichnet, dass $R_2$, $R_4$, $R_5$ und $R_7$ ein Wasserstoffatom bedeuten.

9. Verfahren nach einem der Ansprüche 1 bis 8, dadurch gekennzeichnet, dass $R_1$, $R_6$ und $R_8$ gleichfalls ein Wasserstoffatom bedeuten.

10. Verfahren nach einem der Ansprüche 1 bis 9, dadurch gekennzeichnet, dass $R_3$ eine 2 bis 8 Kohlenstoffatome umfassende Alkylgruppe darstellt.

**Claims for the Contracting States: BE CH DE FR GB IT LI LU NL SE**

1. Acyl chlorides, characterised in that they correspond to the formula

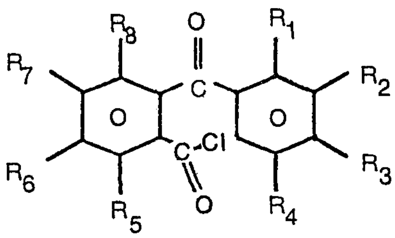

(I)

in which the substituents $R_1$, $R_2$, $R_4$, $R_5$, $R_6$, $R_7$ and $R_8$ represent a hydrogen atom or a carbon containing group containing from 1 to 12 carbon atoms, it being possible for $R_1$, $R_2$, $R_4$, $R_5$, $R_6$, $R_7$ and $R_8$ to be identical or different, in which $R_3$ represents a carbon containing group containing at least 2 carbon atoms, and in which the carbon containing groups are alkyl, alkenyl, aryl, alkylaryl, alkenylaryl, aralkyl, aralkenyl, cycloalkyl or cycloalkenyl groups which can contain, in their structure, at least one hetero-atom consisting of a halogen, oxygen, nitrogen or sulphur.

2. Acyl chlorides according to Claim 1, characterised in that $R_2$, $R_4$, $R_5$ and $R_7$ represent a hydrogen atom.

3. Acyl chlorides according to Claim 2, characterised in that $R_1$, $R_6$ and $R_8$ also represent a hydrogen atom.

4. Acyl chlorides according to any one of Claims 1 to 3, characterised in that $R_3$ represents an alkyl group containing from 2 to 8 carbon atoms.

5. Process for the manufacture of acyl chlorides according to any one of Claims 1 to 4, characterised in that the corresponding substituted ortho-benzoylbenozic acid is reacting with a chlorinating agent.

6. Process according to Claim 5, characterised in that the chlorinating agent used is chosen from amongst phosphorus chlorides and oxychlorides and thionyl chloride.

7. Process according to Claim 5 or 6, characterised in that the reaction is carried out at a temperature of between 250 and 350 K.

8. Process for the manufacture of acyl chlorides according to any one of Claims 1 to 4, characterised in that the corresponding optionally substituted orthophtalic acid chloride is reacted with the corresponding substituted benzene compound, in the presence of a catalyst of the Friedel-Crafts type.

9. Process according Claim 8, characterised in that aluminium chloride is used as the catalyst.

10. Process according to one or other of Claims 8 and 9, characterised in that at least 1 mol of catalyst is used per mol of ortho-phtalic acid chloride.

11. Process according to any one of Claims 8 to 10, characterised in that the reaction is carried out at a temperature of not more than 350 K.

**Claims for the Contracting State: AT**

1. Process of the manufacture of acyl chlorides corresponding to the formula

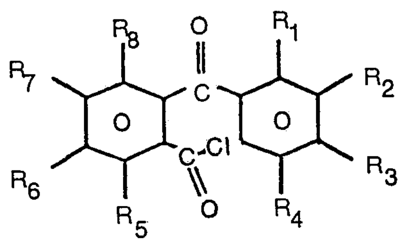

(I)

in which the substituents $R_1$, $R_2$, $R_4$, $R_5$, $R_6$, $R_7$ and $R_8$ represent a hydrogen atom or a carbon containing group containing from 1 to 12 carbon atoms, it being possible for $R_1$, $R_2$, $R_4$, $R_5$, $R_6$, $R_7$ and $R_8$ to be identical or different, in which $R_3$ represents a carbon containing group containing at least two carbon atoms, and in which the carbon containing groups are alkyl, alkenyl, aryl, alkylaryl, alkenylaryl, aralkyl, aralkenyl, cycloalkyl or cycloalkenyl groups which can contain, in their structure, at least one hetero-atom consisting of a halogen, oxygen, nitrogen or sulphur, characterised in that the corresponding substituted ortho-benzoylbenzoic acid is reacted with a chlorinating agent.

2. Process according to Claim 1, characterised in that the chlorinating agent is chosen from amongst phosphorus chlorides and oxychlorides and thionyl chloride.

3. Process according to Claim 1 or 2, characterised in that the reaction is carried out at a temperature of between 250 and 350 K.

4. Process for the manufacture of acyl chlorides corresponding to the formula

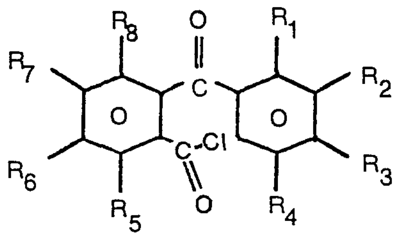

(I)

in which the substituents $R_1$, $R_2$, $R_4$, $R_5$, $R_6$, $R_7$ and $R_8$ represent a hydrogen atom or a carbon containing group containing from 1 to 12 carbon atoms, it being possible for $R_1$, $R_2$, $R_4$, $R_5$, $R_6$, $R_7$ and $R_8$ to be identical or different, in which $R_3$ represents a carbon containing group containing at least 2 carbon atoms, and in which the carbon containing groups are alkyl, alkenyl, aryl, alkylaryl, alkenylaryl, aralkyl, aralkenyl, cycloalkyl or cycloalkenyl groups which can contain, in their structure, at least one hetero-atom consisting of a halogen, oxygen, nitrogen or sulphur characterised in that the corresponding optionally substituted orthophthalic acid chloride is reacted with the corresponding substituted benzene compound, in the presence of a catalyst of the Friedel-Crafts type.

5. Process according to Claim 4, characterised in that aluminium chloride is used as the catalyst.

6. Process according to Claims 4 or 5, characterised in that at least 1 mol of catalyst is used per mol of ortho-phtalic acid chloride.

7. Process according to any one of Claims 4 to 6, characterised in that the reaction is carried out at a temperature of not more than 350 K.

8. Process according to any one of Claims 1 to 7, characterised in that $R_2$, $R_4$, $R_5$ and $R_7$ represent a hydrogen atom.

9. Process according to any one of Claims 1 to 8, characterised in that $R_1$, $R_6$ and $R_8$ also represent a hydrogen atom.

10. Process according to any one of Claims 1 to 9, characterised in that $R_3$ represents an alkyl group containing from 2 to 8 carbon atoms.